# FASCICULE DE BREVET EUROPEEN

(11) **EP 2 240 152 B1**
(45) Date de publication et mention de la délivrance du brevet: **15.08.2018**
(21) Numéro de dépôt: 09704059.6
(22) Date de dépôt: 12.01.2009
(51) Int. Cl.: A61K 8/38, A61K 8/67, A61K 8/73, A61K 31/192, A61K 31/327, A61K 47/36, A61Q 7/00, A61Q 19/08, A61Q 19/00

(54) **COMPOSITION COMPRENANT UN RETINOIDE ET DU PEROXYDE DE BENZOYLE DISPERSE**
ZUSAMMENSETZUNG MIT EINEM RETINOID UND DISPERGIERTEM BENZOYLPEROXID
COMPOSITION CONTAINING A RETINOID AND DISPERSED BENZOYLE PEROXIDE

(30) Priorité: 10.01.2008 FR 0850131
(43) Date de publication de la demande: 20.10.2010
(73) Titulaire: Galderma Research & Development, 06410 Biot (FR)
(72) Inventeur: LOUIS, Fabienne, F-06270 Villeneuve-Loubet (FR); SEGURA, Sandrine, F-06410 Biot (FR); WILLCOX, Nathalie, F-06520 Magagnosc (FR)
(74) Mandataire: Casalonga
(86) Numéro de dépôt international: PCT/FR2009/050040
(87) Numéro de publication internationale: WO 2009/092954

(56) Documents cités:
- WO-A-2007/092312
- WO-A-2008/087354
- FR-A- 2 718 018
- US-A1- 2003 170 196

## Description

L'invention se rapporte à une composition comprenant, dans un milieu physiologiquement acceptable, au moins un rétinoïde, du peroxyde de benzoyle dispersé, au moins un carraghénane et au moins un cation.

L'utilisation de plusieurs classes de principes actifs est un outil thérapeutique auquel on a recours fréquemment, notamment pour le traitement de désordres dermatologiques.
En effet, il est connu d'utiliser les peroxydes, les vitamines D et les rétinoïdes pour le traitement topique de diverses pathologies liées à la peau ou aux muqueuses, en particulier l'acné.
La combinaison de plusieurs traitements locaux (antibiotiques, rétinoïdes, peroxydes, zinc) est également utilisée en dermatologie pour permettre d'augmenter l'efficacité des principes actifs et de diminuer leur toxicité (Cunliffe W.J., J. Dermatol. Treat., 2000, 11 (Suppl. 2), S13-S14).
L'application multiple de différents produits dermatologiques peut être assez lourde et astreignante pour le patient.
On comprend donc l'intérêt de chercher à obtenir un nouveau traitement efficace sur les affections dermatologiques dans une composition stable offrant une bonne cosméticité, permettant une application unique et une utilisation agréable pour le patient.
Parmi cette panoplie de thérapeutiques proposée à l'homme du métier, rien ne l'encourageait à associer, dans la même composition, le peroxyde de benzoyle, un rétinoïde et plusieurs gélifiants

En effet, la formulation d'une telle composition pose plusieurs problèmes.

Tout d'abord, l'efficacité du peroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir l'efficacité optimale du peroxyde de benzoyle, il est important de prévenir sa décomposition avant utilisation, c'est-à-dire durant le stockage. Or, le peroxyde de benzoyle est un composé chimique instable ce qui rend difficile sa formulation dans des produits finis.
La solubilité et la stabilité du peroxyde de benzoyle ont été étudiées par Chellquist et al. dans l'éthanol, le propylène glycol et différents mélanges de polyéthylène glycol 400 (PEG 400) et d'eau (Chellquist E.M. et Gorman W.G., Pharm. Res., 1992, Vol 9: 1341-1346). Il s'avère que le peroxyde de benzoyle est particulièrement soluble dans le PEG400 et l'éthanol.

Ce document précise par ailleurs que la stabilité du peroxyde de benzoyle est fortement influencée par la composition chimique de la formulation et par la température de stockage. Le peroxyde de benzoyle est extrêmement réactif et se dégrade en solution à basse température en raison de l'instabilité de sa liaison peroxyde.
Les auteurs constatent ainsi que le peroxyde de benzoyle en solution se dégrade plus ou moins rapidement dans tous les solvants étudiés en, fonction du type de solvant et de sa concentration.
Les temps de dégradation du peroxyde de benzoyle dans le PEG 400 (0,5 mg/g), dans l'éthanol et dans le propylène glycol sont respectivement de 1,4, 29 et 53 jours à 40°C. Une telle dégradation ne permet pas la préparation d'un produit destiné à la vente.

Il est connu par ailleurs que le peroxyde de benzoyle est plus stable dans l'eau et le propylène glycol lorsqu'il est en suspension (i.e. sous forme dispersée), puisqu'il n'est pas dégradé après 90 jours de conservation dans ces solvants.
Ainsi, pour limiter le problème d'instabilité rapide du peroxyde de benzoyle en solution, il s'est avéré avantageux de formuler le peroxyde de benzoyle sous forme dispersée. Cependant, ce type de formulation n'est pas totalement satisfaisant dans la mesure où on constate toujours une dégradation du peroxyde de benzoyle dans le produit fini.

Une autre difficulté à surmonter pour la préparation d'une composition comprenant à la fois du peroxyde de benzoyle et un rétinoïde est que la plupart des rétinoïdes sont particulièrement sensibles à l'oxydation naturelle, à la lumière visible et aux ultra-violets. Le peroxyde de benzoyle étant un oxydant fort, la compatibilité chimique de ces deux composés dans une même formulation pose de nombreux problèmes de stabilité du point de vue physique et chimique.
Une étude de stabilité de deux rétinoïdes a été réalisée en combinant deux produits commercialisés, l'un contenant un rétinoïde (trétinoine ou adapalène) et le second à base de peroxyde de benzoyle (B. Martin et al., Br.J.Dermatol. (1998) 139, (suppl. 52), 8-11).
La présence de peroxyde de benzoyle dans la formulation provoque une dégradation très rapide des rétinoïdes sensibles à l'oxydation, on mesure que 50% de la trétinoine se dégrade en 2 heures, et 95% en 24 heures. Dans la composition contenant comme rétinoïde l'adapalène, aucune dégradation de l'adapalène n'a été mesurée pendant 24 heures.
Cette étude confirme que le peroxyde de benzoyle se dégrade et dégrade les rétinoïdes sensibles à l'oxydation au cours du temps en relarguant progressivement de l'acide benzoïque dans les produits finis.

Or, il est clair que la dégradation du peroxyde de benzoyle et des rétinoïdes n'est pas souhaitable dans la mesure où elle nuit à l'efficacité de la composition les contenant.

Rien n'incitait donc à associer ces deux agents actifs afin d'obtenir une composition stable de type gel ou émulsion sachant qu'il était usuellement connu que la présence du peroxyde de benzoyle déstabilisait chimiquement et physiquement ces types de compositions.

En particulier, la formulation en gel du peroxyde de benzoyle et d'un rétinoïde est avantageuse pour les traitements topiques, tels que celui de l'acné, car elle évite notamment de laisser subsister un toucher gras sur la peau.

Plus précisément, on entend par gel un système comportant au moins une phase (en général une ou deux phases) thermodynamiquement stable, résultant de la coagulation en un réseau tridimensionnel d'une solution colloïdale. Plus spécifiquement, un gel aqueux correspond à une composition contenant, dans une phase aqueuse, une masse visco-élastique formée à partir de suspensions colloïdales (carraghénane ou association d'un carraghénane avec un autre gélifiant).

En particulier, la formulation en émulsion « légère » du peroxyde de benzoyle et d'un rétinoïde est avantageuse pour les traitements topiques, tels que celui de l'acné, car dans le cas d'une émulsion « légère » elle apporte de l'émollience tout en évitant de laisser subsister un toucher trop gras sur la peau.

Par émulsion « légère », on entend une émulsion contenant une faible proportion de phase grasse, la phase aqueuse restant majoritaire. Une émulsion est un système comportant deux fluides insolubles ou peu solubles l'un dans l'autre, et dans lequel un des fluides se disperse dans l'autre en particules microscopiques. De préférence, les émulsions utilisées comprennent ou non au moins un émulsionnant, une phase hydrophile, de préférence aqueuse, polaire et une phase grasse non polaire. De préférence, elles se présentent sous forme d'émulsions « huile dans l'eau » (H/E), ou « eau dans l'huile » (E/H).

Ainsi, la demande internationale WO 20081087354 décrit des compositions comprenant un rétinoïde et du péroxyde de benzoyle dispersé, qui se présentent sous forme de gel-crème.

Or, une autre difficulté à surmonter pour la préparation d'une composition comprenant notamment du peroxyde de benzoyle, lorsqu'elle se trouve sous forme de gel et d'émulsion, est que les gélifiants de la phase aqueuse sont déstabilisés par l'acide benzoïque libéré lors de la dégradation du peroxyde de benzoyle.
En effet, les gélifiants de la phase aqueuse les plus couramment utilisés avec du peroxyde de benzoyle sont les polymères d'acide acrylique (Carbomer).

Or, l'utilisation de carbomères dans des compositions de type gel aqueux ne donne pas de bons résultats en terme de stabilité chimique du peroxyde de benzoyle et en terme de stabilité rhéologique. Comme décrit par Bollinger (Bollinger, Journal of Pharmaceutical Science, 1977, vol 5), il a été observé une perte de 5 à 20% de peroxyde de benzoyle au bout de 2 mois à 40°C selon le neutralisant du carbomère utilisé. De plus, la libération d'acide benzoïque provoque la dépolymérisation des carbomères, donnant une chute de viscosité pouvant entraîner un déphasage.

Cette instabilité des gels (entant que tel ou en tant que phase aqueuse gélifiée d'une émulsion) de peroxyde de benzoyle nuit donc à leur efficacité et à leur cosméticité.

Par ailleurs, un produit fini, en particulier lorsqu'il s'agit de compositions pharmaceutiques ou cosmétiques, doit conserver tout au long de sa durée de vie, des critères physico-chimiques précis permettant de garantir sa qualité pharmaceutique et cosmétique. Parmi ces critères, il est nécessaire que les propriétés rhéologiques soient conservées. Elles définissent le comportement et la texture de la composition lors de l'application, mais aussi les propriétés de libération du principe actif [Rapport commission SFSTP 1998] et l'homogénéité du produit lorsque les principes actifs y sont présents à l'état dispersé.

Il existe donc le besoin de disposer d'une composition de type gel ou émulsion physiquement et chimiquement stable contenant du peroxyde de benzoyle et un rétinoïde.

Or, la Demanderesse a réalisé de façon surprenante des compositions de type gel et émulsion satisfaisant ce besoin, qui comprend du peroxyde de benzoyle dispersé, libre ou encapsulé, au moins un rétinoïde, au moins un carraghénane et au moins un cation, ayant une bonne stabilité physique, c'est-à-dire ne présentant pas de chute de viscosité dans le temps et notamment à température ambiante, et maintenant une bonne stabilité chimique des deux actifs (peroxyde de benzoyle et rétinoïde). En particulier, on ne constate pas de dégradation des actifs dans le temps et/ou à température ambiante.

L'invention se rapporte donc à une composition comprenant, dans un milieu même physiologiquement acceptable, au moins :
- un rétinoïde,
- du peroxyde de benzoyle
- entre 0,5 et 2% en poids d'au moins un gélifiant de la famille des carraghénanes,
- et entre 0,05 et 0,2% en poids d'au moins un cation,
caractérisée en ce que ladite composition contient au moins un carraghénane choisi parmi les formes kappa et iota et en ce que ladite composition contient le cation calcium.
De préférence, ledit rétinoide et ledit peroxyde de benzoyle étant sous une forme dispersée dans ladite composition.

Selon un mode de réalisation préféré, la composition est une combinaison dont les principes actifs sont associés à des doses fixes au sein d'un seul et même véhicule (formule unique) les délivrant ensemble. De préférence, la composition pharmaceutique sous forme de combinaison fixe est un gel ; dans ce cas, les deux principes actifs sont dispersés et intimement mélangés, lors de la fabrication, dans un seul et même véhicule, qui les délivre ensemble lors de l'application du gel.

Par milieu physiologiquement acceptable, on entend un milieu compatible avec la peau, les muqueuses et les phanères.

De préférence la composition pharmaceutique est destinée à une seule application topique par jour.

Par actif sous forme dispersée selon l'invention, on entend un principe actif sous forme de particules solides, mises en suspension dans un véhicule donné. De telles particules ont notamment une taille supérieure à 10 µm.
De façon avantageuse, la granulométrie du rétinoïde et du peroxyde de benzoyle est telle qu'au moins 80% en nombre des particules, et de préférence au moins 90% en nombre des particules, ont un diamètre inférieur à 25 µm et au moins 99% en nombre des particules ont un diamètre inférieur à 100 µm.

La composition selon l'invention contient au moins un rétinoïde. Par rétinoïde, on entend tout composé se liant aux récepteurs RAR et/ou RXR. Préférentiellement, le rétinoïde est un composé choisi dans la famille des rétinoïdes benzonaphtaléniques (connus également comme dérivés de l'acide naphtoique) tels que décrits dans la demande de brevet EP 0 199 636, et notamment :
L'acide 6-(3-méthylphényl)-2-naphtoïque et son ester méthylique,
L'acide 6-(4-tertiobutylphényl)-2-naphtoïque et son ester méthylique,
L'acide 6-(3-tertiobutylphényl)-2-naphtoïque et son ester méthylique,
L'acide 6-(3,4-diméthoxyphényl)-2-naphtoïque et son ester méthylique,
L'acide 6-(p-(1-adamantylthio)phényl)-2-naphtoïque et son ester méthylique,
L'acide 6-(3-(1-adamantyl)-4-méthoxyphényl)-2-naphtoïque (adapalène) et son ester méthylique,
L'ester méthylique de l'acide 6-[3-(1-adamantyl)-4-tert-butyldiméthylsilyloxyphényl)-2-naphtoïque,
L'ester méthylique de l'acide 6-[3-(1-adamantyl)-4-hydroxyphényl)-2-naphtoïque,
L'acide 6-[3-(1-adamantyl)-4-hydroxyphényl)-2-naphtoïque,
L'ester méthylique de l'acide 6-[3-(1-adamantyl)-4-décyloxyphényl)-2-naphtoïque,
L'acide 6-[3-(1-adamantyl)-4-décyloxyphényl)-2-naphtoïque,
L'ester méthylique de l'acide 6-[3-(1-adamantyl)-4-hexyloxyphényl)-2-naphtoïque,
L'acide 6-[3-(1-adamantyl)-4-hexyloxyphényl)-2-naphtoïque,
L'ester méthylique de l'acide 6-[3-(1-adamantyl)-4-méthoxyphényl)-4-acétoxy-1-méthyl-2-naphtoïque,
L'acide 6-[3-(1-adamantyl)-4-méthoxyphényl)-4-hydroxy-1-méthyl-2-naphtoïque,
L'ester méthylique de l'acide 6-[3-(1-adamantyl)-4-méthoxyphényl)-4-hydroxy-1-méthyl-2-naphtoïque,
L'ester méthylique de l'acide 6-[3-(1-adamantyl)-4-méthoxyphényl)-1-méthyl-2-naphtoïque,
L'acide 6-[3-(1-adamantyl)-4-méthoxyphényl)-1-méthyl-2-naphtoïque,
Le 6-[3-(1-adamantyl)-4-méthoxyphényl)-2-naphtalène méthanol,
L'éthylamide de l'acide 6-[3-(1-adamantyl)-4-méthoxyphényl)-2-naphtoïque,
Le morpholide de l'acide 6-[3-(1-adamantyl)-4-méthoxyphényl)-2-naphtoïque,
L'ester méthylique de l'acide 6-[3-tert-butyl-4-méthoxyphényl)-2-naphtoïque,
L'acide 6-[3-tert-butyl-4-méthoxyphényl)-2-naphtoïque,
L'ester méthylique de l'acide 6-[3-(1,1-diméthyldécyl)-4-méthoxyphényl)-2-naphtoïque,
L'acide 6-[3-(1,1-diméthyldécyl)-4-méthoxyphényl)-2-naphtoïque.

L'objet de l'invention se rapporte donc a une composition comprenant au moins un dérivé d'acide naphtoïque.

L'acide naphtoïque est un composé de formule :

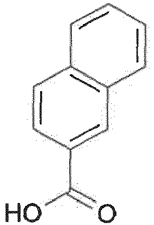

Par dérivé de l'acide naphtoïque, on entend les composés de formule (I): 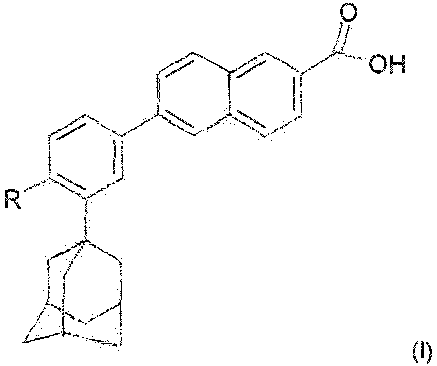 où R représente un atome d'hydrogène, un radical hydroxyle, un radical alkyle, ramifié ou non, ayant de 1 à 4 atomes de carbone, un radical alkoxy ayant de 1 à 10 atomes de carbone ou un radical cycloaliphatique substitué ou non.

Par radical alkyle linéaire ou ramifié ayant de 1 à 4 atomes de carbone, on entend de préférence les radicaux méthyle, éthyle, propyle et butyle.

Par radical alkoxy ayant de 1 à 10 atomes de carbone, on entend de préférence les radicaux méthoxy, éthoxy, propoxy, butoxy, hexyloxy et décyloxy.

Par radical cycloaliphatique, on entend de préférence les radicaux mono ou polycyclique tel que le radical methyl-1 cyclohexyle ou le radical 1-adamantyle.

Parmi les dérivés de l'acide naphtoïque susceptibles d'entrer dans les compositions selon l'invention, on choisira avantageusement l'acide 6-[3-(1-adamantyl)-4-methoxyphenyl]-2-naphtoïque (adapalène), l'acide 6-[3-(1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

Les dérivés de l'acide naphtoïque précités se présentent généralement sous une forme dispersée dans la composition selon l'invention. Les dérivés de l'acide naphtoïque insolubles sont ainsi répartis de façon homogène dans la composition selon l'invention.
En particulier, on préférera l'adapalène ainsi que ses sels.
On entend par sels de l'adapalène, les sels formés avec une base pharmaceutiquement acceptable, notamment des bases minérales telles que la soude, la potasse et l'ammoniaque ou des bases organiques telles que la lysine, l'arginine, la N-méthyl-glucamine.
On entend également par sels de l'adapalène les sels formés avec des amines grasses telles que la dioctylamine et la stéarylamine.

D'autres rétinoïdes peuvent être choisis parmi la tretinoine, l'isotretinoine, l'acide rétinoique, le retinal, le retinol, le retinyl palmitate notamment ceux décrits dans les brevets ou demandes de brevet suivants : US 4,666,941, US 4,581,380, EP 0 210 929, EP 0 232 199, EP 0 260 162, EP 0 292 348, EP 0 325 540, EP 0 359 621, EP 0 409 728, EP 0 409 740, EP 0 552 282, EP 0 584 191, EP 0 514 264, EP 0 514 269, EP 0 661 260, EP 0 661 258, EP 0 658 553, EP 0 679 628, EP 0 679 631, EP 0 679 630, EP 0 708 100, EP 0 709 382, EP 0 722 928, EP 0 728 739, EP 0 732 328, EP 0 740 937, EP 0 776 885, EP 0 776 881, EP 0 823 903, EP 0 832 057, EP 0 832 081, EP 0 816 352, EP 0 826 657, EP 0 874 626, EP 0 934 295, EP 0 915 823, EP 0 882 033, EP 0 850 909, EP 0 879 814, EP 0 952 974, EP 0 905 118, EP 0 947 496, WO98/56783, WO99/10322, WO99/50239, WO99/65872.

Bien entendu, la quantité des deux agents actifs, peroxyde de benzoyle et rétinoïde, dans la composition selon l'invention dépendra de l'association choisie et donc particulièrement du rétinoïde considéré et de la qualité du traitement désiré.
Les concentrations en rétinoïde préférées sont comprises entre 0,0001 et 20 % en poids par rapport au poids total de la composition.

Dans les compositions selon l'invention, les dérivés de l'acide naphtoïque sont utilisés à des concentrations inférieures ou égales à 10 % en poids par rapport au poids total de la composition, et de préférence sont comprises entre 0,001% et 10% en poids par rapport au poids total de la composition et, préférentiellement entre 0,01% et 5%, plus préférentiellement, entre 0,05% et 2%, et tout préférentiellement de 0,1% à 0,3% en poids par rapport au poids total de la composition.

Dans l'ensemble du présent texte, à moins qu'il ne soit spécifié autrement, il est entendu que lorsque des intervalles de concentrations sont donnés, ils incluent les bornes supérieures et inférieures dudit intervalle.

De façon avantageuse, le dérivé de l'acide naphtoïque utilisé dans les compositions selon l'invention est l'acide 6-[3-(1-adamantyl)-4-methoxyphenyl]-2-naphtoïque (adapalène). De manière préférée, dans le cas de l'adapalène, la composition selon l'invention comprend entre 0.001% et 5% et avantageusement entre 0.01% et 1 % en poids d'adapalène par rapport au poids total de la composition, préférentiellement entre 0.01% et 0.5%, de préférence, entre 0.1% et 0.4 % en poids d'adapalène, encore plus préférentiellement à 0.1% ou à 0.3 % en poids d'adapalène.

Dans les compositions selon l'invention, le peroxyde de benzoyle est utilisé à des concentrations allant de 0.5% à 10 % en poids, plus particulièrement de 1% à 7% en poids, encore plus préférentiellement de 2,5% à 5 % en poids par rapport au poids total de la composition.

Le peroxyde de benzoyle pourra aussi bien être utilisé sous la forme libre ou bien sous une forme encapsulée sous forme adsorbée sur, ou absorbée dans, tout support poreux.
Il peut s'agir par exemple - de peroxyde de benzoyle encapsulé dans un système polymérique constitué de microsphères poreuses, comme par exemple des microéponges vendues sous le nom de Microsponges P009A Benzoyle peroxyde par la société Cardinal Healthcare.

Pour donner un ordre de grandeur, la composition selon l'invention comprend avantageusement entre 0,0001 et 20 % en poids de peroxyde de benzoyle et entre 0,0001 et 20 % en poids de rétinoïde par rapport au poids total de la composition, et de préférence, respectivement, entre 0,025 et 10 % en poids de peroxyde de benzoyle et entre 0,001 et 10 % en poids de rétinoïde par rapport au poids total de la composition.

Par exemple, dans les compositions pour le traitement de l'acné, le peroxyde de benzoyle est utilisé, de préférence, à des concentrations allant de 0,5 à 10 % en poids et plus particulièrement de 1,0 à 5 % en poids par rapport au poids total de la composition ; le rétinoïde est quant à lui utilisé dans ce type de composition à des concentrations allant généralement de 0,05 à 1 % en poids par rapport au poids total de la composition.

De façon avantageuse, la granulométrie du rétinoïde et du peroxyde de benzoyle est telle qu'au moins 80% en nombre des particules, et de préférence au moins 90% en nombre des particules, ont un diamètre inférieur à 25 µm et au moins 99% en nombre des particules ont un diamètre inférieur à 100 µm.

La composition selon l'invention comprend en outre au moins un gélifiant de la famille des carraghénanes.

Les carraghénanes sont des polysaccharides constituant les parois cellulaires de diverses algues rouges (Rhodophycées) appartenant aux familles de Gigartinacae, Hypneaceae, Furcellariaceae et Polyideaceae. Ils sont généralement obtenus par extraction aqueuse à partir de souches naturelles desdites algues. Ils comportent des longues chaînes galactanes, polyélectrolytes anioniques. Ces polymères linéaires, formés par des motifs disaccharides, sont composés par deux unités D-galactopyranoses liées alternativement par des liaisons α et β. Ce sont des polysaccharides très sulfatés (20-50%) et les résidus α-D-galactopyranosyles peuvent être sous forme 3',6'-anhydro.

Initialement, on a subdivisé les carraghénanes en deux familles suivant leur solubilité dans le KCl. Les fractions solubles dans le KCI ont été désignées par les préfixes "Kappa", tandis que les termes "Lambda" ont été réservés à celles insolubles. Plus tard, les classifications ont été basées sur le nombre, la position de groupements sulfates ainsi que la présence de pont 3',6'-anhydro sur les résidus β-D-galactopyranosyles. Ceci a abouti aux quatre grandes familles : κ, λ, β, ω, ι.

Les carraghénanes se composent essentiellement de sels de potassium, de sodium, de magnésium, de triéthanolamine et/ou de calcium et d'esters sulfates de polysaccharides.

Ainsi, les carraghénanes sont capables de conférer une viscosité suffisante à la composition pour maintenir en suspension le rétinoïde et le peroxyde de benzoyle, même sous l'influence notamment d'une variation de pH due au relargage d'acide benzoïque par le peroxyde de benzoyle. Pour les formes Kappa et iota, l'apport d'ions potassium ou d'ions calcium est nécessaire pour assurer la gélification et donc impacter la viscosité. En effet, le mécanisme de gélification présente 2 étapes majeures *(*Selim Kara, « Photon Transmission study on swelling of kappa-carrageenan gels prepared in various concentrations », International Journal of Biological Macromolecules, 33 (2003), 235-243*) (*Tommasina Coviello, « Polysaccharide hydrogels for modified release formulations », Journal of Controlled release, 119 (2007), 5-24*):*
- la formation d'hélices ;
- l'action des cations induit le rapprochement des hélices et la formation d'agrégats. Le mécanisme de gélification a donc lieu.

La quantité de carraghénane est comprise entre 0.5% et 2% en poids, par rapport au poids total de la composition, et en particulier 0.5%, 1% et 2%.

Les carrhaghénanes sont notamment commercialisés par la société IMCD sous le nom de Gelcarin® et de Viscarin® (par exemple : Gelcarin GP812N®, Gelcarin GP379NF®, Viscarin GP209NF®).

Les compositions de la présente invention peuvent se présenter sous toutes les formes galéniques normalement utilisées pour une application topique, notamment sous forme de dispersions aqueuses, huileuses, de suspensions, de gels aqueux, anhydres ou lipophiles, d'émulsions (lotions, crèmes ou crèmes sans émulsionnant) de consistance liquide , semi-solide ou solide, obtenues par dispersion d'une phase grasse dans une phase aqueuse (H/E) ou inversement (E/H) avec ou sans émulsionnant.

De préférence, les compositions selon l'invention se présentent sous forme d'émulsions (crèmes, lotions, crèmes sans émulsionnant), de suspensions, de gels, et plus préférentiellement sous la forme de gels et d'émulsions.

L'homme du métier veillera à choisir les excipients constituant les compositions selon l'invention en fonction de la forme galénique souhaitée et de manière à ce que les propriétés avantageuses de la composition selon l'invention soient respectées.

La composition de type gel selon l'invention peut en outre notamment comprendre un ou plusieurs des ingrédients suivants :
a) un ou plusieurs agents gélifiants additionnel et/ou agents de suspension et/ou agents gélifiants pH-indépendants,
b) un ou plusieurs agents émollients et/ou humectants ;
c) un ou plusieurs agents mouillants ;
d) optionnellement, un ou plusieurs additifs.

La composition de type émulsion (crème, lotion, crème sans émulsionnant) selon l'invention peut en outre notamment comprendre un ou plusieurs des ingrédients suivants :
a) un ou plusieurs agents gélifiants additionnels et/ou agents de suspension et/ou agents gélifiants pH-indépendants,
b) un ou plusieurs agents émollients et/ou humectants ;
c) un ou plusieurs agents mouillants,
d) un ou plusieurs excipients lipophiles composant la phase grasse
e) optionnellement un ou plusieurs émulsionnants
f) optionnellement, un ou plusieurs additifs.

La composition selon l'invention pourra contenir au moins un agent gélifiant additionnel et/ou agent de suspension et/ou agent gélifiant pH-indépendant distinct des carraghénanes.. L'autre gélifiant additionnel et/ou agent de suspension et/ou agent gélifiant pH-indépendant peut en particulier être choisi dans le groupe formé par les celluloses et ses dérivés, les gommes de polysaccharides, les silicates, les gélifiants de la famille des polyacrylamides,, la famille des polymères acryliques couplés à des chaînes hydrophobes, et la famille des amidons modifiés.

Parmi les celluloses et ses dérivés, on trouve à titre d'exemple la microcrystalline cellulose et carboxymethyl cellulose de sodium vendue sous le nom d'Avicel CL-611® par la société FMC Biopolymer, l'éthylcellulose vendu sous le nom d'Ethocel® par la société Dow Chemical, l'hydroxypropylméthylcellulose vendu sous le nom de Methocel E4M® par la société Dow Chemical, l'hydroxyéthylcellulose vendu sous le nom de Natrosol 250HHX® par la société Aqualon, la carboxyméthylcellulose vendu sous le nom de Blanose® par la société Aqualon.

Les gommes de polysaccharides sont des mélanges complexes de plusieurs polysaccharides de hauts poids moléculaires obtenus par exsudation de certaines plantes. Parmi les différents types de gommes polysaccharides, on trouve à titre d'exemple non limitatif, la gomme arabique, la gellan gum, la gomme adragante et la gomme de xanthane, entre autre commercialisée par la société CP Kelco sous le nom de Xantural® (par exemple : Xantural® 180).

Par silicate, on entend en particulier des dérivés d'argile, plus spécifiquement des silicates d'aluminium et de magnésium. Ces composés sont notamment commercialisés par la société R.T. Vanderbilt sous le nom de Veegum® (par exemple : Veegum® HV ou Veegum® K).

Parmi les gélifiants de type polyacrylamide, on peut citer notamment le mélange Sodium acryloyldimethyltaurate copolymer / isohexadecane polysorbate 80 vendu sous le nom de Sepineo P600® (ou Simulgel 600PHA®) par la société Seppic, le mélange polyacrylamide / isoparaffine C13-14 / laureth-7 comme, par exemple, vendu sous le nom de Sepigel 305® par la société Seppic.

Parmi les polymères acryliques couplés à des chaînes hydrophobes, on peut citer notamment le PEG-150/decyl/SMDI copolymer vendu sous le nom de Aculyn 44® (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)) vendu par la société Rohm&Haas.

Parmi les gélifiants de la famille des amidons modifiés, on peut citer notamment l'amidon de pomme de terre modifié vendu sous le nom de Structure Solanace® ou bien leurs mélanges par la société National Starch.

La quantité de gélifiant additionnel est généralement comprise entre 0,01% et 20% en poids par rapport au poids total de la composition.

On préfère les associations de carrhaghénanes avec les polyacrylamides de préférence le mélange Sodium acryloyldimethyltaurate copolymer / isohexadecane polysorbate 80 vendu sous le nom de Simulgel 600PHA® ou de carrhaghénanes avec les celluloses et ses dérivés, de préférence l'hydroxyéthylcellulose vendu sous le nom Natrosol 250HHX® ou des carrhaghénanes avec les gommes de polysaccharides.

Selon un mode particulier, la composition peut comprendre un carrhaghénane en association avec au moins un polyacrylamide et au moins un gélifiant cellulosique et ses dérivés.

La quantité de polyacrylamide peut varier dans une large mesure et dépend en particulier de la viscosité désirée, du carraghénane utilisé et éventuellement du et/ou des autres gélifiants présents dans la composition. Pour donner un ordre de grandeur, le Simulgel 600PHA peut être présent dans la composition selon l'invention dans des quantités comprises entre 0,01 à 10 %, de manière préférentielle allant de 0,05 à 6 % en poids par rapport au poids total de la composition. Avantageusement, la quantité est comprise entre 2 et 3 %, comme par exemple 2% ou 3%, par rapport au poids total de la composition.

La quantité de gomme de polysaccharides peut varier dans une large mesure et dépend en particulier de la viscosité désirée, du carraghénane utilisé et éventuellement du et/ou des autres gélifiants présents dans la composition. Pour donner un ordre de grandeur, la gomme de xanthane peut être présente dans la composition selon l'invention dans des quantités comprises entre 0,01 à 5 %, de manière préférentielle allant de 0,05 à 2 % en poids par rapport au poids total de la composition. Avantageusement, la quantité est comprise entre 0,1 et 1 %, comme par exemple 0,2% ou 0,5%, par rapport au poids total de la composition.

La quantité de gélifiant cellulosique peut varier dans une large mesure et dépend en particulier de la viscosité désirée, du carraghénane utilisé et éventuellement du et/ou des autres gélifiants présents dans la composition. Pour donner un ordre de grandeur, le gélifiant cellulosique peut être présent dans la composition selon l'invention dans des quantités comprises entre 0,01 et 10%, de manière préférentielle allant de 0,05 à 5% en poids par rapport au poids total de la composition. Avantageusement, la quantité est comprise entre 0,5 et 2%, comme par exemple 0,5% par rapport au poids total de la composition.

La composition comprend au moins un cation qui a pour rôle d'assurer la gélification et donc d'impacter la viscosité de la formulation. A titre d'exemple, on peut citer les ions potassium et les ions calcium vendus notamment sous le nom de calcium chloride dihydrate par la société Sigma Aldrich.

Le cation calcium est présent dans la composition selon la présente invention.

La quantité de cation est comprise entre 0,05 et 0,2% en poids par rapport au poids total de la composition, comme par exemple 0,05%, 0,1% et 0,2% par rapport au poids total de la composition.

La composition peut comprendre un ou plusieurs humectants et/ou émollients. A titre d'exemple, on peut citer la glycérine et le sorbitol, les sucres (à titre d'exemple le glucose, le lactose), les polyéthylènes glycol (à titre d'exemple le Lutrol E400), l'urée, les acides aminés (à titre d'exemple la sérine, la citrulline, l'arginine, l'asparagine, l'alanine).

L'agent humectant et/ou émollient particulièrement préféré est la glycérine.

Parmi les agents mouillants qui ont pour rôle de diminuer la tension superficielle et de permettre un plus grand étalement du liquide, on utilise préférentiellement, sans que cette liste soit limitative, un agent mouillant pouvant présenter préférentiellement une HLB de 10 à 14, sans que cette liste soit limitative, des composés de la famille des Poloxamers et/ou glycols et plus particulièrement le Synperonic PE/L44, et/ou le Synperonic PE/L62 et/ou des composés de la famille des glycols tels que le propylène glycol, le dipropylène glycol, le lauroglycol, le propylène glycol dipélargonate, l'éthoxydiglycol. De préférence, les agents mouillants sont sous forme liquide de manière à s'incorporer aisément dans la composition sans qu'il soit nécessaire de la chauffer.

L'agent mouillant particulièrement préféré est le propylène glycol et le Synperonic PE/L44 vendu par la société Uniqema.

La composition selon l'invention peut comprendre optionnellement un ou plusieurs émulsionnants.

Les émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.

Le pouvoir émulsifiant des émulsionnants non-ioniques est étroitement lié à la polarité de la molécule. Cette polarité est définie par la HLB (Balance Hydrophile/Lipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des tensioactifs hydrophiles.

Les émulsionnants peuvent être classés, selon leur structure, sous les termes génériques "ioniques" (anioniques, cationiques, amphotères) ou "non ioniques". Les émulsionnants non ioniques sont des émulsionnants qui ne se dissocient pas en ions dans l'eau et sont donc insensibles aux variations de pH.

On peut citer, à titre d'exemples non limitatifs des émulsionnants non ioniques présentant une HLB élevée, les esters de sorbitan tels que le POE(20) sorbitan monooléate, vendu sous le nom de Tween 80® (HLB=15) ; le POE(20) sorbitan monostéarate vendu sous le nom de Tween 60® (HLB=14.9) ; les éthers d'alcools gras tels que le POE (21) stéaryl ether (HLB= 15.5) vendu sous le nom de Brij 721® par la société Uniqema, ou le ceteareth 20 vendu sous le nom de Eumulgin B2® (HLB de 15,5) par la société Cognis, les esters de polyoxyethylene glycol tel que le glyceryl stearate and PEG 100 stéarate vendu sous le nom de Arlacel 165 FL® (HLB=11) par la société Uniqema , le PEG 6 Stéarate et PEG 32 stéarate vendu sous le nom de TEFOSE 1500® (HLB= 10) par la société Gateffossé, les sucroesters de haut HLB tel que le PEG 20 methyl glucose sesquistéarate vendu sous les noms de glucamate SSE20® (HLB=15) par la société Amerchol, le sucrose laurate vendu sous le nom de Surfhope C-1216® (HLB=16) et de Surfhope SE Pharma D-1216® par la société Gattefossé, le sucrose stéarate vendu sous le nom de Surfhope C-1811® (HLB=11), le Surfhope SE Pharma D1811® (HLB=11), et le Surfhope SE Pharma D1816® (HLB=16) par la société Gattefossé, le sucrose palmitostéarate vendu sous le nom de Surfhope SE Pharma D-1616® par la société Gattefossé, , les esters de polyglycérol. De préférence, lesdits émulsionnants non ioniques de haute HLB, présentent une HLB comprise entre 10 et 18.

On citera, comme exemples non limitatifs les émulsionnants non ioniques de basse HLB (lipophiles), les esters de sorbitan, tels que le monostéarate de sorbitan (HLB = 4.7) vendu sous le nom de Span 60 par la société Uniqema, les esters de glycérol tel que le monostéarate de glycerol vendu sous le nom de Cutina GMSVPH (HLB=3.8) par la sociéte Cognis, les esters de polyethylène glycol tel que le PEG-6 isostéarate vendu sous le nom de Olepal isostéarique® (HLB=8) par la société Gattefossé, les sucroesters de bas HLB tel que le methyl glucose sesquistéarate vendu sous le nom de Glucate SS® (HLB= 6) par la société Amerchol et le sucrose dilaurate vendu sous le nom de Surfhope C-1205® (HLB=5) et le sucrose tristéarate vendu sous le nom de Surfhope C-1803® (HLB=3) et le Surfhope Pharma D-1803® (HLB=3) par la société Gattefossé.

On peut aussi citer comme autres agents émulsionnants non-ioniques, des cires auto-émulsionnantes qui permettent d'obtenir des émulsions stables facilement par simple dispersion à chaud. A titre d'exemple, le cetearyl alcohol (and) polysorbate 60 vendu sous le nom de Polawax NF par la société Croda, la Polawax GP200 vendu par la société Croda.

De préférence, on utilisera, en tant que système émulsionnant un ou plusieurs couples "émulsionnant non ionique de haute HLB" / "émulsionnant non ionique de faible HLB", il pourra en particulier s'agir d'un système émulsionnant non ionique comprenant au moins un émulsionnant non ionique présentant une HLB supérieure à environ 10 et au moins un émulsionnant non ionique présentant une HLB inférieure à environ 10.

Le ratio de chacun des deux émulsionnants formants le couple précité est déterminé le plus souvent par le calcul de la HLB requise de la phase grasse utilisée.

A titre d'émulsionnants préférés on peut citer :
- des émulsionnants hydrophiles de type, Glyceryl Stéarate & PEG-100 Stéarate vendu sous le nom Arlacel 165FL® par la société Uniqema; le PEG 6 stéarate et PEG 32 stéarate vendu sous le nom de TEFOSE 1500® par Gattefossé, le PEG 20 methyl glucose sesquistéarate vendu sous le nom de Glucamate SSE 20® par Amerchol, le sucrose laurate vendu sous le nom de Surfhope SE Pharma D-1216® par la société Gattefossé, le sucrose stéarate vendu sous le nom de Surfhope SE Pharma D1816® par la société Gattefossé, le sucrose palmitostéarate vendu sous le nom de Surfhope SE Pharma D-1616® par la société Gattefossé, le Polyoxyethylene (21) Stéaryl Ether vendu sous le nom Brij721® par Uniqema, le Ceteareth 20 vendu sous le nom d'Eumulgin B2PH® par Cognis, les esters de sorbitan vendu sous le nom de Tween 80® et Tween 60®.
- des émulsionnants lipophiles de type methyl glucose sesquistéarate tels que le Glucate SS® vendu par Amerchol, le sucrose dilaurate tel que le Surfhope C-1205® vendu par Gattefossé et le sucrose tristéarate tel que le Surfhope D-1803® vendu par Gattefossé.

La composition selon l'invention peut également comprendre une phase grasse. Cette phase grasse peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale ou leur substitut d'origine végétale, on peut citer l'huile d'amande douce, l'huile de palme, l'huile de soja, l'huile de sésame, l'huile de tournesol.

Comme huile animale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglyceride tel que Miglyol 812® vendu par la société Univar.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning.

Comme polyisobutene hydrogéné, on peut citer les Parleam® vendus par la société Rossow.

Comme alcool de Guerbet, on peut citer l'octyldodecanol vendu sous le nom d'Eutanol G par la société Cognis.

On pourra également mettre des corps gras solides tel que des cires naturelles ou synthétiques, des acides gras tel que l'acide stéarique, des alcools gras tel que le Speziol C18 pharma vendu par la société Cognis, et des agents de texture de type tribehenate, tel que le Compritol 888 vendu par la société Gattefossé ou les huiles de ricin hydrogénées tel que le Cutina HR vendu par la société Cognis. Dans ce cas, l'homme du métier adaptera la température de chauffage de la préparation en fonction de la présence ou non de ces solides.

Pour la composition selon l'invention, l'huile de silicone et plus particulièrement la ST-Cyclomethicone 5NF® est préférée.

La composition peut comprendre optionnellement tout additif usuellement utilisé dans le domaine cosmétique ou pharmaceutique, tel que des antioxydants, des filtres solaires, des conservateurs (à titre d'exemple le chlorure de benzalkonium, le phénoxyéthanol, l'alcool benzylique, la diazolidinylurée, les parabens, ou leurs mélanges), des charges, des électrolytes, des colorants, un stabilisant du péroxyde de benzoyle (à titre d'exemple le docusate de sodium, le sodium C14-16 oléfine sulfonate, acide lactique, acide citrique), des agents neutralisants de type bases ou acides usuels, minéraux ou organiques (à titre d'exemple la triethanolamine, la solution de soude 10%, le tampon acide citrique/sodium citrate, le tampon acide succinique/succinate de sodium), des parfums, des huiles essentielles, des actifs cosmétiques, des hydratants, des vitamines, des acides gras essentiels, des sphingolipides, des composés auto-bronzants tels que la DHA, des agents apaisants et protecteurs de la peau tels que l'allantoïne.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à une teneur comprise entre 0 à 20 % en poids par rapport au poids total de la composition, de préférence comprise entre 0 et 10% en poids.

La phase aqueuse selon l'invention peut comprendre de l'eau, une eau florale telle que l'eau de bleuet, ou une eau thermale ou minérale naturelle comme par exemple l'eau de Vittel.

Ladite phase aqueuse peut être présente à une teneur comprise entre 10 et 95 % en poids par rapport au poids total de la composition, de préférence comprise entre 20 et 90 % en poids.

En particulier, l'invention concerne également une composition pharmaceutique ou cosmétique pour application topique sur la peau, les phanères ou les muqueuses, sous forme d'un gel aqueux, caractérisée en ce qu'elle contient, dans un milieu physiologiquement acceptable et compatible avec l'application topique sur la peau, les phanères ou les muqueuses, (exprimé en pourcentage en poids) :
- 20 à 90% d'eau ;
- 0,01 à 10 %, préférentiellement 0,1 à 8 % d'agents mouillants ;
- 0,0001 à 20 %, préférentiellement 0,025 à 10 %, de peroxyde de benzoyle ;
- 0,0001 à 20 %, préférentiellement 0,001 à 10 %, de rétinoïde ;
- 0,5 à 2% de carraghénanes ;
- 0,05 à 0,2% de cations ;
- 0,01 à 15%, préférentiellement 0,05 à 10% d'un ou plusieurs humectants et/ou émollients ;
- 0,01 à 10%, préférentiellement 0,05 à 6% de gélifiant additif et/ou agent de suspension et/ou gélifiant pH-indépendant ;
- 0 à 20%, préférentiellement 0 à 10% d'additifs.

En particulier, l'invention concerne également une composition pharmaceutique ou cosmétique pour application topique sur la peau, les phanères ou les muqueuses, sous forme d'une émulsion, caractérisée en ce qu'elle contient, dans un milieu physiologiquement acceptable et compatible avec l'application topique sur la peau, les phanères ou les muqueuses (exprimé en pourcentage en poids) :
- 20 à 90% d'eau ;
- 0,01 à 10 %, préférentiellement 0,1 à 8 % d'agents mouillants ;
- 0,0001 à 20 %, préférentiellement 0,025 à 10 %, de peroxyde de benzoyle ;
- 0,0001 à 20 %, préférentiellement 0,001 à 10 %, de rétinoïde ;
- 0,5 à 2% de carraghénanes ;
- 0,05 à 0,2% de cations ;
- 0,01 à 15%, préférentiellement 0.05 à 10% d'un ou plusieurs humectants et/ou émollients ;
- 0,01 à 10%, préférentiellement 0,05 à 6% de gélifiant additionnel et/ou agent de suspension et/ou gélifiant pH-indépendant ;
- 0 à 15%, préférentiellement 0 à 10% d'émulsionnants,
- 0 à 20%, préférentiellement 0 à 10% d'additifs.

Les méthodes de préparation de la composition selon l'invention sont des méthodes classiques éventuellement adaptables par l'homme du métier.

Le procédé principal de préparation de la composition selon l'invention comprend à titre d'exemple les étapes suivantes :

### Etape a: préparation de la phase active 1:

Mélange de l'eau purifiée et du principe actif 1 (adapalène) avec au moins un agent mouillant jusqu'à ce que ledit dérivé d'acide naphtoique soit parfaitement dispersé, afin d'obtenir la phase active 1 ;

### Etape b: préparation de la phase active 2:

Mélange de l'eau purifiée et du principe actif 2 (peroxyde de benzoyle), avec au moins un agent mouillant jusqu'à ce que ledit peroxyde de benzoyle soit parfaitement dispersé, afin d'obtenir la phase active 2 ;

### Etape c : Préparation de la phase aqueuse

Dans un bêcher, on introduit sous agitation, si nécessaire à chaud, de l'eau purifiée, le gélifiant de la famille des carraghénanes, le ou les gélifiants additionnels et/ou gélifiants agents de suspension et/ou agents pH indépendants (à l'exception du polyacrylamide), le cation, le ou les humectants et/ou émollients et optionnellement, le ou les conservateurs, le ou les émulsionnants hydrophiles.

### Etape d: le mélange des phases actives

Les deux phases actives obtenues respectivement en a) et b) sont mélangées. L'agitation est maintenue jusqu'à parfaite homogénéisation.

### Etape e (optionnellement pour l'obtention d'une émulsion): Préparation de la phase grasse :

Mélange des composés huileux, des corps gras solides et optionnellement des émulsionnants lipophiles, des conservateurs.

Le mélange est chauffé et après homogénéisation, le silicone volatile est introduit en dernier si présent dans la composition

### Etape f (optionnelle): Emulsification :

A chaud, la phase grasse est introduite dans la phase aqueuse afin de réaliser l'émulsification. Le chauffage est maintenu quelques minutes, puis la préparation est refroidie.

### Etape g: addition de la phase active unique

Introduction de la phase active unique obtenue en d) dans la phase aqueuse obtenue en c) pour les gels ou dans la phase obtenue en f) pour les émulsions.

### Etape h (optionnelle) : Addition du polyacrylamide

On introduit sous agitation le polyacrylamide à la phase obtenue en g). L'agitation est maintenue jusqu'à parfaite homogénéité.

### Etape i: Neutralisation :

L'agent de neutralisation du gélifiant est introduit si nécessaire, dans la phase obtenue à l'étape g).

### Etape j : (optionnelle) ajustement en eau

Si nécessaire, un ajustement en eau est réalisé.

Le procédé alternatif de préparation de la composition selon l'invention comprend à titre d'exemple les étapes suivantes:
Les principes actifs sont mélangés dans la 1^{ère} étape du procédé décrit ci-dessus ; ainsi les étapes a) et b) sont remplacées par l'étape a') :
a') préparation de la phase active unique comprenant les deux actifs.
Le procédé est ensuite poursuivi comme décrit à partir de l'étape c) avec suppression de l'étape d).

De manière plus détaillée, le procédé principal de préparation de la composition selon l'invention comprend les étapes suivantes:

### Etape a: préparation de la phase active 1:

Dans un bêcher, on introduit sous agitation de l'eau purifiée, le principe actif (Adapalène), les agents mouillants (type Synperonic PE/L62, Synperonic PE/L44, propylène glycol). On laisse sous agitation jusqu'à parfaite dispersion.

### Etape b: préparation de la phase active 2:

Dans un bêcher, on introduit sous agitation de l'eau purifiée, le principe actif (peroxyde de benzoyle), les agents mouillants (type Synperonic PE/L62, Synperonic PE/L44, propylène glycol). On laisse sous agitation jusqu'à parfaite dispersion.

### Etape c : Préparation de la phase aqueuse

Dans un bêcher, on introduit sous agitation, si nécessaire à chaud, de l'eau purifiée, le gélifiant de la famille des carraghénanes (type Gelcarin GP379NF), le ou les gélifiants additionnels (type Xantural 180, Natrosol 250HHX) et/ou agents de suspension (type Avicel CL-611) et/ou gélifiants pH indépendants (à l'exception du Simulgel 600PHA), le cation, le ou les émollients et/ou humectants (type glycerine) et optionnellement le ou les conservateurs (type méthyle paraben), les émulsionnants hydrophiles (type Tween 80).

### Etape d: le mélange des phases actives

Les deux phases actives obtenues respectivement en a) et b) sont mélangées, l'agitation est maintenue jusqu'à parfaite homogénéisation ;

### Etape e (optionnelle): Préparation de la phase grasse :

Mélange des huiles et corps gras solides (type olépal isostéarique, Cetiol SN PH, Crodamol DA, Speziol C18, Cosbiol) et optionnellement des émulsionnants type (Glucate SS, Glucamate SSE 20, Brij 721, Téfose 1500), des conservateurs (type phénoxyéthanol et propyle paraben).
Le mélange est chauffé et après homogénéisation, le silicone volatile (type Cyclométhicone 5NF) est introduit si ce dernier est présent dans la composition.

### Etape f (optionnelle): Emulsification :

A chaud, la phase grasse est introduite dans la phase aqueuse afin de réaliser l'émulsification. Le chauffage est maintenu quelques minutes, puis la préparation est refroidie.

### Etape g: addition de la phase active unique

Introduction de la phase active unique obtenue en e) dans la phase aqueuse obtenue en c) pour les gels ou dans la phase obtenue en f) pour les émulsions.

### Etape h (optionnelle) : Addition du Simulgel 600PHA

On introduit sous agitation le Simulgel 600PHA à la phase obtenue en g). L'agitation est maintenue jusqu'à parfaite homogénéité.

### Etape i : Neutralisation :

Si nécessaire, l'agent de neutralisation du gélifiant (type triéthanoloamine ou solution d'hydroxyde de sodium à 10%) est introduit dans la phase obtenue à l'étape g).

### Etape j : (optionnelle) ajustement en eau

Si nécessaire, un ajustement en eau est réalisé.

De manière plus détaillée, le procédé alternatif de préparation de la composition selon l'invention comprend les étapes suivantes:
Les principes actifs sont mélangés dans la 1^{ère} étape du procédé décrit ci-dessus ; ainsi les étapes a) et b) sont remplacées par l'étape a') :
a') Préparation de la phase active unique comprenant les deux actifs :
On mélange le dérivé d'acide naphtoïque, le peroxyde de benzoyle avec au moins un agent mouillant, dans de l'eau, jusqu'à ce que ledit peroxyde de benzoyle et ledit dérivé d'acide naphtoïque soient parfaitement dispersés afin d'obtenir une phase active unique selon les mêmes conditions opératoires.

Le procédé est ensuite poursuivi comme décrit à partir de l'étape c) et l'étape d) est supprimée.

La présente invention a aussi pour objet la composition telle que décrite précédemment à titre de médicament.

L'invention se rapporte également à l'utilisation de la nouvelle composition telle que décrite précédemment en cosmétique et en dermatologie.

De part l'activité kératolytique, bactéricide et anti-inflammatoire du peroxyde de benzoyle et l'activité marquée des rétinoïdes dans les domaines de la différentiation et de la prolifération cellulaire, les compositions de l'invention conviennent particulièrement bien dans les domaines thérapeutiques suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle, l'hidradenite supurative,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que les folliculites,
4) pour traiter toutes les proliférations dermiques ou épidermiques qu'elles soient bénignes ou malignes, qu'elles soient ou non d'origine virale telles que verrues vulgaires, les verrues planes, le molluscurn contagiosum, et l'épidermodysplasie verruciforme, les papillomatoses orales ou florides et les proliférations pouvant être induites par les ultra- violets notamment dans le cas des kératoses actiniques,
5) pour réparer ou lutter contre le vieillissement de la peau, qu'il soit photo-induit ou chronologique, ou pour réduire les pigmentations, ou toutes pathologies associées au vieillissement chronologique ou actinique,
6) pour traiter de manière préventive ou curative les troubles de la cicatrisation, les ulcères cutanés, pour prévenir ou pour réparer les vergetures, ou encore pour favoriser la cicatrisation,
7) pour lutter contre les troubles de la fonction sébacée tels que Phyperséborrhée de l'acné ou la séborrhée simple,
8) dans le traitement de toute affection d'origine fongique au niveau cutané tel que le tinea pedis et le tinea versicolor,
9) dans le traitement d'affections dermatologiques à composante immunologique,
10) dans le traitement de désordres cutanés dus à une exposition aux rayonnements U.V.

Les compositions selon l'invention sont particulièrement adaptées au traitement, de manière préventive ou curative, des acnés vulgaires.

Un objet de l'invention se rapporte également à la fabrication de préparation pharmaceutique destinée à la prévention ou au traitement des affections dermatologiques liées à des désordres de la différentiation et/ou de la prolifération cellulaire et/ou de la kératinisation, ainsi qu'à la fabrication de préparation pharmaceutique destinée à prévenir ou à traiter les acnés vulgaires.

La présente invention va maintenant être illustrée au moyen des exemples suivants ainsi que des données de stabilité physique et chimique présentées ci-dessous sans toutefois en limiter sa portée.

La stabilité physique des formulations est contrôlée par une observation macroscopique et microscopique de la formulation conservée à température ambiante à T0, T+1mois, T+2mois et T+3mois.
A TA, l'observation macroscopique permet de garantir l'intégrité physique des produits.
L'observation microscopique permet d'évaluer la qualité de la dispersion des deux actifs. L'adapalène est observé en lumière fluorescente alors que le peroxyde de benzoyle est observé en lumière polarisée.
On complète la caractérisation du produit fini par une mesure du seuil d'écoulement et de la viscosité.

Pour la mesure du seuil d'écoulement, un rhéomètre HAAKE de type VT550 avec un mobile de mesure SVDIN a été utilisé.
Les rhéogrammes sont réalisés à 25°C et en vitesse imposée de 0 à 100s⁻¹. Les valeurs de viscosité sont données aux valeurs de cisaillement de 4 s⁻¹, 20s⁻¹, 100s⁻¹ (γ). Par seuil d'écoulement (τ₀ exprimé en Pascal) on entend la force nécessaire (contrainte de cisaillement minimum) pour vaincre les forces de cohésion de type Van der Waals et provoquer l'écoulement.

Pour les mesures de viscosité, on utilise des viscosimètres de type Brookfield RVDVII+ et LDVDII+.
Les gammes de viscosité mesurables avec les 2 types de Brookfield sont les suivantes :
Viscosimètre RVDVII+ : 100 cP - 40 McP
Viscosimètre LVDVII+/ 15 cP - 6 McP
Pour les émulsions, on considère que l'on a à l'instant initial T0 :
Une crème si la viscosité est supérieure à 30 000 cP
Une lotion si la viscosité est inférieure à 30 000 cP (Lucinda Bushe, ACPS October 22, 2003 Pharmaceutical nomenclature-Issue and challenges).

La stabilité chimique de l'adapalène et du peroxyde de benzoyle est assurée par un dosage HPLC.

Les résultats sont exprimés en mg/g d'adapalène et de peroxyde de benzoyle, et en % par rapport au titre théorique.

Les exemples ci-dessous permettent d'illustrer l'invention, sans toutefois en limiter sa portée.

### Exemple 1 : Formulation de type gel contenant du peroxyde de benzoyle 2.5%, de l'Adapalène à 0.1% et le Gelcarin GP 379NF à 0.5% en tant que gélifiant de la famille des carraghénanes :

| **Exemple 1** | |
|---|---|
| Eau | QS 100% |
| Adapalene | 0,1% |
| Peroxyde de benzoyle | 2,5% |
| Propylène glycol | 4.0% |
| Synperonic PE/L44 | 0,2% |
| Glycerine | 4.0% |
| Gelcarin GP379NF | 0.5% |
| Calcium Chloride dihydrate | 0.05% |
| Simulgel 600PHA | 3.0% |

### Données de stabilités :

**➢ Stabilité physique :**

| **Caractérisation à T0** | | |
|---|---|---|
| Aspect macroscopique | | Gel blanc |
| Aspect microscopique | | Dispersion des actifs sans agrégats > 100µm |
| pH | | 5.45 |
| Données de viscosité | Haake (4s⁻¹/20s⁻¹/100s-¹) | 93/129/185 |
| | Brookfield RVDVII+ (S29 ; 5rpm) | 66560cP |

| | | T+1mois | T+2mois | T+3mois |
|---|---|---|---|---|
| Aspect macroscopique | TA | Identique à T0 | Identique à T0 | Identique à T0 |
| Aspect microscopique | TA | Identique à T0 | Identique à T0 | Identique à T0 |
| pH | TA | 4.87 | 4.80 | 4.34 |
| Rhéologie Haake (4s⁻¹/20s⁻¹/100s⁻¹) | | 104/144/200 | 99/135/197 | 97/139/196 |
| Brookfield RVDVII+ (S29 ; 5rpm) | | 71800cP | 69400cP | 73800cP |

### ➢ Stabilité chimique

**- Adapalène :**

| Conditions de stabilité | Temps | T0 | T+1mois | T+2mois | T+3mois |
|---|---|---|---|---|---|
| TA | mg/g | 0.93 | 0.91 | 0.94 | 0.92 |
| | % du titre théorique | 93 | 91 | 94 | 92 |

**- Peroxyde de benzoyle:**

| Conditions de stabilité | Temps | T0 | T+1mois | T+2mois | T+3mois |
|---|---|---|---|---|---|
| TA | mg/g | 23.30 | 23.46 | 23.38 | 23.34 |
| | % du titre théorique | 93 | 94 | 94 | 93 |

### Exemple 2 : Formulation de type gel contenant du peroxyde de benzoyle 2.5%, de l'Adapalène à 0.1% et le Gelcarin GP 379NF à 0.50% en tant que gélifiant de la famille des carraghénanes :

| **Exemple 2** | |
|---|---|
| Eau | QS 100% |
| Adapalene | 0,1% |
| Peroxyde de benzoyle | 2,5% |
| Propylène glycol | 4.0% |
| Synperonic PE/L44 | 0,2% |
| Glycerine | 4.0% |
| Gelcarin GP379NF | 0.50% |
| Calcium Chloride dihydrate | 0.05% |
| Simulgel 600PHA | 2.0% |
| Natrosol 250HHX | 0.5% |

### Données de stabilités :

**➢ Stabilité physique :**

| **Caractérisation à T0** | | |
|---|---|---|
| Aspect macroscopique | | Gel blanc |
| Aspect microscopique | | Dispersion des actifs sans agrégats > 100µm |
| pH | | 6.24 |
| Données de viscosité | Haake (s⁻¹/20s⁻¹/100s⁻¹) | 185/237/300 |
| | Brookfield RVDVII+ (S29: 5rpm) | 130.10³cP |

| | | T+1mois | T+2mois | T+3mois |
|---|---|---|---|---|
| Aspect macroscopique | TA | Identique à T0 | Identique à T0 | Identique à T0 |
| Aspect microscopique | TA | Identique à T0 | Identique à T0 | Identique à T0 |
| pH | TA | 4.92 | 4.66 | 4.26 |
| Rhéologie Haake (4s⁻¹120s⁻¹/100s⁻¹) | | 190/254/321 | 170/216/261 | 178/248/327 |
| Brookfield RVDVII+ (S29 ; 5rpm) | | 134.10³cP | 133.10³cP | 136.10³cP |

### Stabilité chimique

**- Adapalène :**

| Conditions de stabilité | Temps | T0 | T+1mois | T+2mois | T+3mois |
|---|---|---|---|---|---|
| TA | mg/g | 0.98 | 0.98 | 1.00 | 1.01 |
| | % du titre théorique | 98 | 98 | 100 | 101 |

**- Peroxyde de benzoyle:**

| Conditions de stabilité | Temps | T0 | T+1mois |
|---|---|---|---|
| TA | mg/g | 22.79 | 24.62 |
| | % du titre théorique | 91 | 99 |

### Exemple 3 : Formulation de type lotion contenant du peroxyde de benzoyle 1.0%, de l'Adapalène à 0.3% et le Gelcarin GP 379NF à 2.00% en tant que gélifiant de la famille des carraghénanes :

| **Exemple 3** | |
|---|---|
| Eau | QS 100% |
| Adapalene | 0,3% |
| Peroxyde de benzoyle | 1.0% |
| Dipropylène glycol | 3.0% |
| Synperonic PE/L44 | 0,2% |
| Glycerine | 4.0% |
| Gelcarin GP379NF | 2.0% |
| Calcium Chloride dihydrate | 0.2% |
| Simulgel 600PHA | 2.0% |
| Cetiol SN PH | 5.0% |
| Perhydrosqualene | 5.0% |
| Nipasol M | 0.05% |
| Arlacel 165FL | 3.0% |
| Brij 721 | 3.0% |

### Données de stabilités :

**➢ Stabilité physique :**

| **Caractérisation à T0** | | |
|---|---|---|
| Aspect macroscopique | | Lotion blanche |
| Aspect microscopique | | Dispersion des actifs sans agrégats > 100µm |
| pH | | 6.21 |
| Données de viscosité | Haake (4s⁻¹/20s⁻¹/100s⁻¹) | 49/89/185 |
| | Brookfield LVDVII+ (S64 ; 10rpm) | 29814cP |

| | | T+1mois | T+2mois | T+3mois |
|---|---|---|---|---|
| Aspect macroscopique | TA | Identique à T0 | Identique à T0 | Identique à T0 |
| Aspect microscopique | TA | Identique à T0 | Identique à T0 | Identique à T0 |
| pH | TA | 6.12 | 5.47 | 4.89 |
| Rhéologie Haake (4s⁻¹/20s⁻¹/100s⁻¹) | | 38/73/170 | 42/80/172 | 43/83/171 |
| Brookfield LVDVII+ (S64 ; 10rpm) | | 28254cP | 31073cP | 28194cP |

### ➢ Stabilité chimique

**- Adapalène :**

| Conditions de stabilité | Temps | T0 | T+1mois | T+2mois | T+3mois |
|---|---|---|---|---|---|
| TA | mg/g | 2.87 | 2.90 | 2.85 | 2.93 |
| | % du titre théorique | 96 | 97 | 95 | 98 |

**- Peroxyde de benzoyle:**

| Conditions de stabilité | Temps | T0 | T+1mois | T+2mois | T+3mois |
|---|---|---|---|---|---|
| TA | mg/g | 9.50 | 9.69 | 9.57 | 9.52 |
| | % du titre théorique | 95 | 97 | 96 | 95 |

### Exemple 4 : Formulation de type crème contenant du peroxyde de benzoyle 2.5%, de l'Adapalène à 0.1% et le Gelcarin GP 379NF à 1.00% en tant que gélifiant de la famille des carraghénanes :

| **Exemple 4** | |
|---|---|
| Eau | QS 100% |
| Adapalène | 0.1% |
| Peroxyde de benzoyle | 2,5% |
| Propylene glycol | 6.0% |
| Synperonic PE/L44 | 0.2% |
| Glycerine | 7.0% |
| Gelcarin GP379NF | 1.0% |
| Calcium Chloride dihydrate | 0.1% |
| Simulgel 600PHA | 2.0% |
| Eumulgin B2PH | 3.0% |
| Speziol C18 Pharma | 2.0% |
| Miglyol 812N | 7.0% |
| ST-Cyclomethicone 5NF | 6.0% |
| Arlacel 165FL | 3.0% |

### Données de stabilités :

**➢ Stabilité physique :**

| **Caractérisation à T0** | | |
|---|---|---|
| Aspect macroscopique | | Crème blanche |
| Aspect microscopique | | Dispersion des actifs sans agrégats > 100µm |
| pH | | 6.33 |
| Données de viscosité | Haake (4s⁻¹/20s⁻¹/100s⁻¹) | 101/137/220 |
| | Brookfield RVDVII+ (S34 ; 5rpm) | 80128cP |

| | | T+1mois | T+2mois | T+3mois |
|---|---|---|---|---|
| Aspect macroscopique | TA | Identique à T0 | Identique à T0 | Identique à T0 |
| Aspect microscopique | TA | Identique à T0 | Identique à T0 | Identique à T0 |
| pH | TA | 5.80 | 4.73 | 5.00 |
| Rhéologie Haake (4s⁻¹/20s⁻¹/100s⁻¹) | | 103/139/209 | 101/136/214 | 107/146/223 |
| Brookfield RVDVII+ (S34 ; 5rpm) | | 81664cP | 67200cP | 76544cP |

### ➢ Stabilité chimique

**- Adapalène :**

| Conditions de stabilité | Temps | T0 | T+1mois | T+2mois | T+3mois |
|---|---|---|---|---|---|
| TA | mg/g | 0.97 | 0.97 | 0.96 | 0.98 |
| | % du titre théorique | 97 | 97 | 96 | 98 |

**- Peroxyde de benzoyle:**

| Conditions de stabilité | Temps | T0 | T+1mois | T+2mois | T+3mois |
|---|---|---|---|---|---|
| TA | mg/g | 23.11 | 23.90 | 23.68 | 23.66 |
| | % du titre théorique | 92 | 96 | 95 | 95 |

## Revendications

1. Composition comprenant, dans un même milieu physiologiquement acceptable, au moins un rétinoide, du peroxyde de benzoyle dispersé, entre 0,5 et 2% en poids d'au moins un gélifiant de la famille des carraghénanes, et entre 0,05 et 0,2% en poids d'au moins un cation, **caractérisée en ce que** ladite composition contient au moins un carraghénane choisi parmi les formes kappa et iota et **en ce que** ladite composition contient le cation calcium.

2. Composition selon la revendication 1, **caractérisée en ce que** le rétinoide est un dérivé de l'acide naphtoique.

3. Composition selon l'une des revendications 1 à 2, **caractérisée en ce qu'**elle contient du calcium chloride dihydrate.

4. Composition selon l'une des revendications précédentes, **caractérisée en ce qu'**elle comprend au moins un gélifiant additionnel.

5. Composition selon la revendication 4, **caractérisée en ce que** le gélifiant additionnel est choisi parmi les celluloses semi-synthétiques, les gommes de polysaccharides, les silicates, les polymères acryliques couplés à des chaînes hydrophobes, les amidons modifiés et les polyacrylamides.

6. Composition selon la revendication 5, **caractérisée en ce que** la quantité de gélifiant additionnel est comprise entre 0,01% et 20% en poids par rapport au poids total de la composition.

7. Composition selon l'une des revendications 1 à 6, **caractérisée en ce qu'**elle comprend entre 0,0001 et 20 % de rétinoïde en poids par rapport au poids total de la composition.

8. Composition selon l'une des revendications 1 à 7, **caractérisée en ce que** le rétinoide ou le dérivé d'acide naphtoique est l'adapalène.

9. Composition selon l'une des revendications 1 à 8, **caractérisée en ce qu'**elle comprend entre 0,0001 et 20 % de peroxyde de benzoyle en poids par rapport au poids total de la composition.

10. Composition selon l'une des revendications 1 à 9, **caractérisée en ce que** le peroxyde de benzoyle est sous forme encapsulée ou libre.

11. Composition selon l'une quelconque des revendications précédentes, **caractérisée en ce qu'**elle comprend un agent propénétrant.

12. Composition selon la revendication 11, **caractérisée en ce qu'**elle comprend entre 0,01 et 20 % d'agent propénétrant en poids par rapport au poids total de la composition.

13. Composition selon la revendication 11 ou 12, **caractérisée en ce que** l'agent propénétrant est le Propylène glycol.

14. Composition selon l'une quelconque des revendications précédentes, **caractérisée en ce qu'**elle comprend un tensioactif liquide mouillant.

15. Composition selon la revendication 14, **caractérisée en ce qu'**elle comprend entre 0,01 et 10% de tensioactif liquide mouillant en poids par rapport au poids total de la composition.

16. Composition selon la revendication 15, **caractérisée en ce que** le tensioactif liquide mouillant est un poloxamer.

17. Composition selon l'une quelconque des revendications précédentes, **caractérisée en ce qu'**elle est sous forme de gel.

18. Composition selon la revendication 17, **caractérisée en ce qu'**elle comprend dans un milieu physiologiquement acceptable (exprimé en pourcentage en poids):
- 20 à 90% d'eau ;
- 0,01 à 10 %, préférentiellement 0,1 à 8 % d'agents mouillants ;
- 0,0001 à 20 %, préférentiellement 0,025 à 10 %, de peroxyde de benzoyle ;
- 0,0001 à 20 %, préférentiellement 0,001 à 10 %, de rétinoïde ;
- entre 0,5 et 2% de carraghénane ;
- 0,05 à 0,2% de cations ;
- 0,01 à 15%, préférentiellement 0,05 à 10% d'un ou plusieurs humectants et/ou émollients ;
- 0,01 à 10%, préférentiellement 0,05 à 6% de gélifiant additif et/ou agent de suspension et/ou gélifiant pH-indépendant ;
- 0 à 20%, préférentiellement 0 à 10% d'additifs.

19. Composition selon l'une quelconque des revendications précédentes, **caractérisée en ce qu'**elle est sous forme d'une émulsion,

20. Composition selon la revendication 19 **caractérisée en ce qu'**elle comprend dans un milieu physiologiquement acceptable (exprimé en pourcentage en poids) :
- 20 à 90% d'eau ;
- 0,01 à 10 %, préférentiellement 0,1 à 8 % d'agents mouillants ;
- 0,0001 à 20 %, préférentiellement 0,025 à 10 %, de peroxyde de benzoyle ;
- 0,0001 à 20 %, préférentiellement 0,001 à 10 %, de rétinoïde ;
- entre 0,5 et 2% de carraghénane ;
- 0,05 à 0,2% de cations ;
- 0,01 à 15%, préférentiellement 0.05 à 10% d'un ou plusieurs humectants et/ou émollients ;
- 0,01 à 10%, préférentiellement 0,05 à 6% de gélifiant additionnel et/ou agent de suspension et/ou gélifiant pH-indépendant ;
- 0 à 15%, préférentiellement 0 à 10% d'émulsionnants,
- 0 à 20%, préférentiellement 0 à 10% d'additifs.

21. Composition selon l'une quelconque des revendications 1 à 20 à titre de médicament.

22. Utilisation d'une composition selon l'une quelconque des revendications 1 à 21 pour la fabrication d'une préparation pharmaceutique destinée à prévenir ou à traiter les affections dermatologiques liées à des désordres de la différentiation et/ou la prolifération cellulaire et/ou de la kératinisation.

23. Utilisation d'une composition selon la revendication 22 pour fabriquer une préparation pharmaceutique destinée à prévenir ou à traiter les acnés vulgaires.

## Patentansprüche

1. Zusammensetzung, die in dem gleichen physiologisch unbedenklichen Medium mindestens ein Retinoid, dispergiertes Benzoylperoxid, zwischen 0,5 und 2 Gew.-% mindestens eines Gelbildners aus der Familie der Carrageenane und zwischen 0,05 und 0,2 Gew.-% mindestens eines Kations umfasst, **dadurch gekennzeichnet, dass** die Zusammensetzung mindestens ein Carrageenan, das aus den kappa- und iota-Formen ausgewählt ist, enthält und dass die Zusammensetzung das Kation Calcium enthält.

2. Zusammensetzung nach Anspruch 1, **dadurch gekennzeichnet, dass** es sich bei dem Retinoid um ein Naphthoesäurederivat handelt.

3. Zusammensetzung nach einem der Ansprüche 1 bis 2, **dadurch gekennzeichnet, dass** sie Calciumchloriddihydrat enthält.

4. Zusammensetzung nach einem der vorhergehenden Ansprüche, **dadurch gekennzeichnet, dass** sie mindestens einen zusätzlichen Gelbildner umfasst.

5. Zusammensetzung nach Anspruch 4, **dadurch gekennzeichnet, dass** der zusätzliche Gelbildner aus halbsynthetischen Cellulosen, Polysaccharid-Gummen, Silikaten, mit hydrophoben Ketten gekoppelten Acrylpolymeren, modifizierten Stärken und Polyacrylamiden ausgewählt ist.

6. Zusammensetzung nach Anspruch 5, **dadurch gekennzeichnet, dass** die Menge an zusätzlichem Gelbildner zwischen 0,01 und 20 Gew.-%, bezogen auf das Gesamtgewicht der Zusammensetzung, liegt.

7. Zusammensetzung nach einem der Ansprüche 1 bis 6, **dadurch gekennzeichnet, dass** sie zwischen 0,0001 und 20 Gew.-% Retinoid, bezogen auf das Gesamtgewicht der Zusammensetzung, umfasst.

8. Zusammensetzung nach einem der Ansprüche 1 bis 7, **dadurch gekennzeichnet, dass** es sich bei dem Retinoid bzw. Naphthoesäurederivat um Adapalen handelt.

9. Zusammensetzung nach einem der Ansprüche 1 bis 8, **dadurch gekennzeichnet, dass** sie zwischen 0,0001 und 20 Gew.-% Benzoylperoxid, bezogen auf das Gesamtgewicht der Zusammensetzung, umfasst.

10. Zusammensetzung nach einem der Ansprüche 1 bis 9, **dadurch gekennzeichnet, dass** das Benzoylperoxid in verkapselter oder freier Form vorliegt.

11. Zusammensetzung nach einem der vorhergehenden Ansprüche, **dadurch gekennzeichnet, dass** sie ein penetrationsförderndes Mittel umfasst.

12. Zusammensetzung nach Anspruch 11, **dadurch gekennzeichnet, dass** sie zwischen 0,01 und 20 Gew.-% penetrationsförderndes Mittel, bezogen auf das Gesamtgewicht der Zusammensetzung, umfasst.

13. Zusammensetzung nach Anspruch 11 oder 12, **dadurch gekennzeichnet, dass** es sich bei dem penetrationsfördernden Mittel um Propylenglykol handelt.

14. Zusammensetzung nach einem der vorhergehenden Ansprüche, **dadurch gekennzeichnet, dass** sie ein benetzendes flüssiges Tensid umfasst.

15. Zusammensetzung nach Anspruch 14, **dadurch gekennzeichnet, dass** sie zwischen 0,01 und 10 Gew.-% benetzendes flüssiges Tensid, bezogen auf das Gesamtgewicht der Zusammensetzung, umfasst.

16. Zusammensetzung nach Anspruch 15, **dadurch gekennzeichnet, dass** es sich bei dem benetzenden flüssigen Tensid um ein Poloxamer handelt.

17. Zusammensetzung nach einem der vorhergehenden Ansprüche, **dadurch gekennzeichnet, dass** sie in Form eines Gels vorliegt.

18. Zusammensetzung nach Anspruch 17, **dadurch gekennzeichnet, dass** sie in einem physiologisch unbedenklichen Medium Folgendes umfasst (ausgedrückt in Gewichtsprozent):
- 20 bis 90 % Wasser;
- 0,01 bis 10 %, vorzugsweise 0,1 bis 8 %, Netzmittel;
- 0,0001 bis 20 %, vorzugsweise 0,025 bis 10 %, Benzoylperoxid;
- 0,0001 bis 20 %, vorzugsweise 0,001 bis 10 %, Retinoid;
- zwischen 0,5 und 2 % Carrageenan;
- 0,05 bis 0,2 % Kationen;
- 0,01 bis 15 %, vorzugsweise 0,05 bis 10 %, eines oder mehrerer Feuchthaltemittel und/oder Emollientien;
- 0,01 bis 10 %, vorzugsweise 0,05 bis 6 %, zusätzlichen Gelbildner und/oder Suspendiermittel und/oder pH-unabhängigen Gelbildner;
- 0 bis 20 %, vorzugsweise 0 bis 10 %, Additive.

19. Zusammensetzung nach einem der vorhergehenden Ansprüche, **dadurch gekennzeichnet, dass** sie in Form einer Emulsion vorliegt.

20. Zusammensetzung nach Anspruch 19, **dadurch gekennzeichnet, dass** sie in einem physiologisch unbedenklichen Medium Folgendes umfasst (ausgedrückt in Gewichtsprozent):
- 20 bis 90 % Wasser;
- 0,01 bis 10 %, vorzugsweise 0,1 bis 8 %, Netzmittel;
- 0,0001 bis 20 %, vorzugsweise 0,025 bis 10 %, Benzoylperoxid;
- 0,0001 bis 20 %, vorzugsweise 0,001 bis 10 %, Retinoid;
- zwischen 0,5 und 2 % Carrageenan;
- 0,05 bis 0,2 % Kationen;
- 0,01 bis 15 %, vorzugsweise 0,05 bis 10 %, eines oder mehrerer Feuchthaltemittel und/oder Emollientien;
- 0,01 bis 10 %, vorzugsweise 0,05 bis 6 %, zusätzlichen Gelbildner und/oder Suspendiermittel und/oder pH-unabhängigen Gelbildner;
- 0 bis 15 %, vorzugsweise 0 bis 10 %, Emulgatoren;
- 0 bis 20 %, vorzugsweise 0 bis 10 %, Additive.

21. Zusammensetzung nach einem der Ansprüche 1 bis 20 als Medikament.

22. Verwendung einer Zusammensetzung nach einem der Ansprüche 1 bis 21 zur Herstellung einer pharmazeutischen Zubereitung zur Prävention oder Behandlung von dermatologischen Leiden, die mit Störungen der Zelldifferenzierung und/oder Zellproliferation oder der Keratinisierung verbunden sind.

23. Verwendung einer Zusammensetzung nach Anspruch 22 zur Herstellung einer pharmazeutischen Zubereitung zur Prävention oder Behandlung von Acne vulgaris.

## Claims

1. Composition comprising, in the same physiologically acceptable medium, at least one retinoid, dispersed benzoyl peroxide, between 0.5 and 2% by weight of at least one gelling agent of the family of carrageenans, and between 0.05 and 0.2% by weight of at least one cation, **characterized in that** said composition contains at least one carrageenan chosen from the kappa and iota forms and **in that** said composition contains the calcium cation.

2. Composition according to Claim 1, **characterized in that** the retinoid is a naphthoic acid derivative.

3. Composition according to either of Claims 1 and 2, **characterized in that** it contains calcium chloride dihydrate.

4. Composition according to one of the preceding claims, **characterized in that** it comprises at least one additional gelling agent.

5. Composition according to Claim 4, **characterized in that** the additional gelling agent is chosen from semisynthetic celluloses, polysaccharide gums, silicates, acrylic polymers coupled to hydrophobic chains, modified starches and polyacrylamides.

6. Composition according to Claim 5, **characterized in that** the amount of additional gelling agent is between 0.01 and 20% by weight, with respect to the total weight of the composition.

7. Composition according to one of Claims 1 to 6, **characterized in that** it comprises between 0.0001 and 20% of retinoid by weight, with respect to the total weight of the composition.

8. Composition according to one of Claims 1 to 7, **characterized in that** the retinoid or the naphthoic acid derivative is adapalene.

9. Composition according to one of Claims 1 to 8, **characterized in that** it comprises between 0.0001 and 20% of benzoyl peroxide by weight, with respect to the total weight of the composition.

10. Composition according to one of Claims 1 to 9, **characterized in that** the benzoyl peroxide is in the encapsulated or free form.

11. Composition according to any one of the preceding claims, **characterized in that** it comprises a propenetrating agent.

12. Composition according to Claim 11, **characterized in that** it comprises between 0.01 and 20% of propenetrating agent by weight, with respect to the total weight of the composition.

13. Composition according to Claim 11 or 12, **characterized in that** the propenetrating agent is propylene glycol.

14. Composition according to any one of the preceding claims, **characterized in that** it comprises a wetting liquid surfacant.

15. Composition according to Claim 14, **characterized in that** it comprises between 0.01 and 10% of wetting liquid surfactant by weight, with respect to the total weight of the composition.

16. Composition according to Claim 15, **characterized in that** the wetting liquid surfactant is a poloxamer.

17. Composition according to any one of the preceding claims, **characterized in that** it is in the form of a gel.

18. Composition according to Claim 17, **characterized in that** it comprises, in a physiologically acceptable medium (expressed as percentage by weight):
- from 20 to 90% of water;
- from 0.01 to 10%, preferably from 0.1 to 8%, of wetting agents;
- from 0.0001 to 20%, preferably from 0.025 to 10%, of benzoyl peroxide;
- from 0.0001 to 20%, preferably from 0.001 to 10%, of retinoid;
- between 0.5 and 2% of carrageenan;
- from 0.05 to 0.2% of cations;
- from 0.01 to 15%, preferably from 0.05 to 10%, of one or more humectants and/or emollients;
- from 0.01 to 10%, preferably from 0.05 to 6%, of additional gelling agent and/or suspending agent and/or pH-independent gelling agent;
- from 0 to 20%, preferably from 0 to 10%, of additives.

19. Composition according to any one of the preceding claims, **characterized in that** it is in the form of an emulsion.

20. Composition according to Claim 19, **characterized in that** it comprises, in a physiologically acceptable medium (expressed as percentage by weight):
- from 20 to 90% of water;
- from 0.01 to 10%, preferably from 0.1 to 8%, of wetting agents;
- from 0.0001 to 20%, preferably from 0.025 to 10%, of benzoyl peroxide;
- from 0.0001 to 20%, preferably from 0.001 to 10%, of retinoid;
- between 0.5 and 2% of carrageenan;
- from 0.05 to 0.2% of cations;
- from 0.01 to 15%, preferably from 0.05 to 10%, of one or more humectants and/or emollients;
- from 0.01 to 10%, preferably from 0.05 to 6%, of additional gelling agent and/or suspending agent and/or pH-independent gelling agent;
- from 0 to 15%, preferably from 0 to 10%, of emulsifiers;
- from 0 to 20%, preferably from 0 to 10%, of additives.

21. Composition according to any one of Claims 1 to 20 as a medicament.

22. Use of a composition according to any one of Claims 1 to 21 in the manufacture of a pharmaceutical preparation intended to prevent or treat dermatological conditions linked to disorders of cell differentiation and/or proliferation and/or of keratinization.

23. Use of a composition according to Claim 22 for manufacturing a pharmaceutical preparation intended to prevent or treat acne vulgaris.
